# EUROPEAN PATENT APPLICATION

(11) **EP 0 882 437 A2**
(43) Date of publication of application: **09.12.1998**
(21) Application number: 98610016.2
(22) Date of filing: 04.06.1998
(51) Int. Cl.: A61F 5/445

(54) **An ostomy appliance with a faceplate having a concave/convex contour**

(30) Priority: 04.06.1997 DK 65797
(71) Applicant: COLOPLAST A/S, 3050 Humlebaek (DK)
(72) Inventor: Nielsen, Inger Mann, 2000 Frederiksberg (DK); Olsen, Eskil Höjland, 2930 Klampenborg (DK); Gothjaelpsen, Laila Busk, 2650 Hvidovre (DK)
(74) Representative: Hegner, Anette

(57) **Abstract**

An ostomy appliance comprising a faceplate with all adhesive wafer for securing the faceplate to the peristomal skin of a user, and a collection pag securable to the faceplate. The adhesive wafer has radially extending tongues and concave contour portions between the tongues.

This arrangement makes the appliance particular useful for attachment to irregular skin areas, and a further advantage is, that skin problems may be reduced.

## Description

### FIELD OF THE INVENTION

The present invention relates to an ostomy appliance.

### BACKGROUND OF THE INVENTION

In connection with surgery for a number of diseases in the gastro-intestinal tract a consequence is, in many cases, that the patient is left with an abdominal stoma such as a colostomy, an ileostomy or a urostomy. In such cases or in connection with a fistula the patient will have to rely on an external ostomy appliance to collect the bodily waste material emerging from such opening.

Ostomy appliances are well known. Such appliances may be two-piece or one-piece appliances. In both types of appliances, a collection bag is attached to the user's abdomen in the region of the stoma for receiving waste material or exudates from the ostomy. With two-piece appliances an adhesive faceplate or body side member is first attached to the user's abdomen, and a receiving member or bag is then attached to the body side ostomy member for receiving waste material from the ostomy. In one-piece appliances a collection bag has an integrated adhesive faceplate for attaching the bag to the user.

When using one-piece appliances, the whole appliance, including the adhesive wafer or pad securing the appliance to the skin is removed and replaced by a fresh appliance. When using two-piece appliances, the body side ostomy member is often left in place for several days, and only the receiving member or bag is replaced.

The service time of the body side ostomy member depends on the amount and the aggressiveness of the exudates and of the tightness of the sealing between the ostomy and the body side ostomy member.

In the known appliances it is necessary to change the body side member of two-piece appliance when the centre part of the adhesive wafer or pad has been sufficiently deteriorated to allow access of the aggressive exudates to the skin surrounding the stoma, irrespective of the fact that the wafer as such has a much longer wearing time. The access of aggressive exudates to the skin is causing skin problems.

Skin problems are common for persons having a stoma. Generally, about 40 % of them have skin problems (Pearl et al. 1985 "Early local complications from intestinal stomas", Arch. Surg. 120; 1145-1147.) and the frequency is especially high for persons having an urostomy or ileostomy. About 80 % of the persons having an ileostomy have skin problems (Hellman, J.D., Lago, C.P. 1990 "Dermatologic complications in colostomy and ileostomy patients", International Journal of Dermatology, 29 (2); 129-133.). The skin problems are mostly pronounced in a circular area about the stoma (10-15 mm from the stoma) (Hellman and Lago 1990).

Frequent changing of the body side member of a two-piece appliance or the frequent exchange of a one-piece appliance is undesirable due to the irritation of the skin, and if the intervals between exchanging of the body side member can be increased, then the nuisance of wearing an ostomy appliance can be reduced and the user's quality of life may consequently be improved.

Many known ostomy appliances have a body side ostomy member or face plate with a circular contour. From patent publications such as US 4 889 534, GB 2 198 954 and EP 686 381 ostomy appliances are known having face plates with substantially square contour with rounded corners, or in other words, faceplates of known ostomy appliance always have a convex contour and possibly with linear portions. Usually, the user will place the face plates with the same orientation on the skin each time he attaches a fresh face plate or complete ostomy appliance, and furthermore, all known ostomy appliances have face plates which are intended to be placed each with the same orientation.

Such known face plates furthermore have a relatively large and substantially plane surface, which can give problems with fitting to curved or irregular skin areas.

It is the object of the invention to alleviate such problems with known ostomy appliances.

### SUMMARY OF THE INVENTION

According to the invention an ostomy appliance is disclosed having radially extending tongues with concave contour portions between the tongues. This makes it possible to obtain a better fit to curved or irregular skin areas, because the tongues can be bent or folded independent of the others to follow the irregular skin areas.

It will also be possible to reduce skin irritation. When changing the ostomy appliance, or at least the faceplate, the user can attach the fresh faceplate with a different angular orientation, i.e. rotated relative to the previous faceplate, whereby the tongues of the fresh faceplate are placed where the concave contour portions between the tongues of the old faceplate were placed, and vice versa. It is thereby obtained that important skin areas are relieved from the above problems of skin irritation, since they will have rest periods, i.e. periods without any faceplate portions attached even at times where the user wears an ostomy appliance.

### BRIEF DESCRIPTION OF THE DRAWINGS

Fig. 1 is a perspective view of an ostomy appliance according to the invention,
Fig. 2 is a sectional drawing of the ostomy appliance in fig. 1 in exploded view, and
Fig. 3 shows the contour of the faceplate used in the ostomy appliance in figs. 1 and 2.

All the drawings are schematic drawings that are not necessarily drawn to scale but are merely intended to illustrate the principles of the invention.

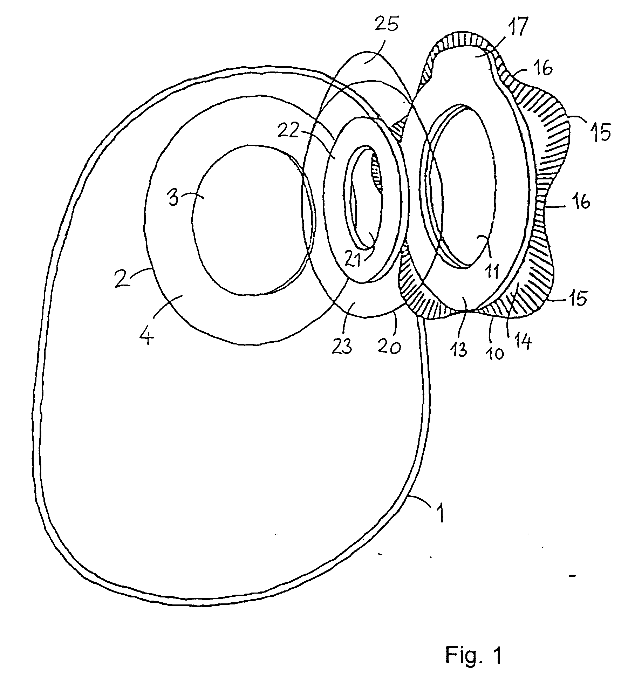

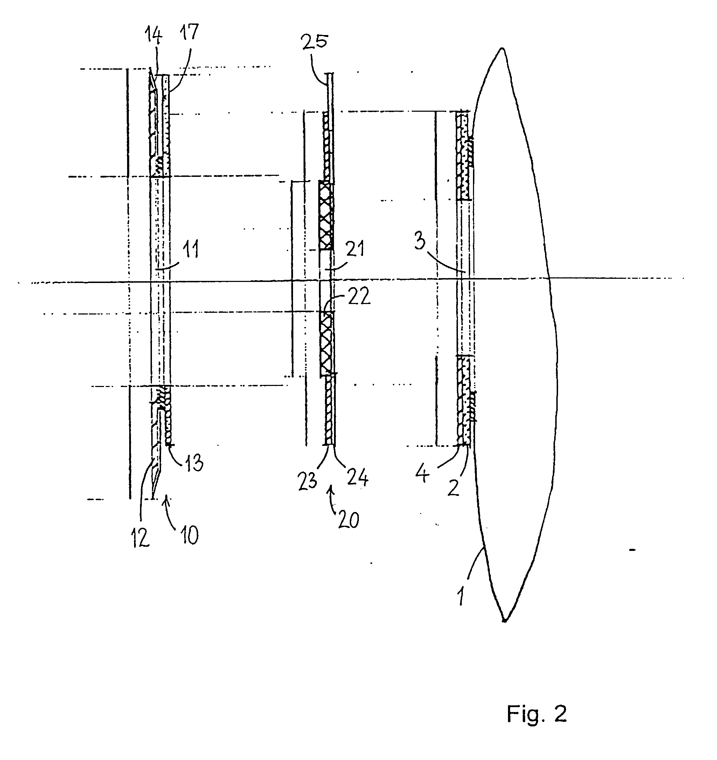

Figures 1 and 2 show an ostomy appliance comprising a collection bag **1**, a body side ostomy member or faceplate **10** and an intermediate sealing member **20**.

The collection bag **1** has a flange **2** with a layer of adhesive **4**, and a bag opening **3** giving access to the interior of the collection bag.

The body side ostomy member or faceplate **10** has a body side member opening **11** therein and a layer of skin friendly adhesive **12** intended for attaching the faceplate **10** to the peristomal skin of a user with the body side member opening **11** receiving the stoma of the user. The faceplate **10** also has a flange **13** secured to a backing layer **14** for the layer of adhesive **12**.

The intermediate sealing member **20** has a central opening **21** therein, a soft, elastically mouldable sealing substance in the form of a ring **22** at the edge of the central opening **21**, and a layer of adhesive **23** around the soft sealing ring **22.** A carrying sheet **24** carries the soft sealing ring **22** and the layer of adhesive **23.**

The ostomy appliance in fig. 1 will be used the following way. First, the user will attach the faceplate **10** to the peristomal skin with the layer of adhesive **12** facing the skin and with the body side member opening **11** receiving his or her stoma. Next, the user will take the intermediate sealing member **20** and attach it to the flange **13** of the faceplate **10** with the soft sealing ring **22** received in the body side member opening **11**. The soft sealing ring **22** can then be shaped or moulded to fit snugly around the stoma, and finally the user will attach the collection bag **1** by means of the adhesive **4** to the carrying sheet **24** of the intermediate sealing member **20,** which will then be situated between the flange **13** of the faceplate **10** and the flange 2 of the collecticn bag **1**. The components of the ostomy appliance are in known manner being held together by means of the adhesive layers **4** and **23** on the contacting surfaces. Alternatively, mechanical coupling or locking mechanisms also known in the art can be used. The soft sealing member or ring **22** will give a tight fit to the user's stoma.

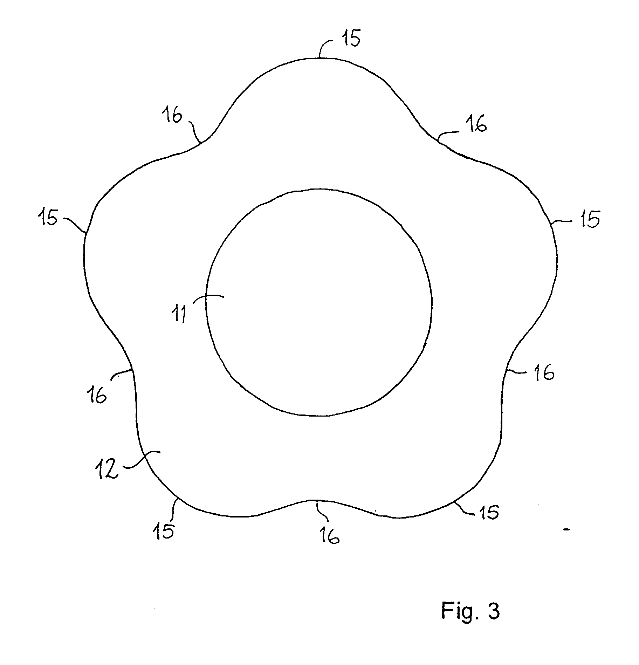

Figure 3 shows the faceplate **10** seen from the side with the layer of adhesive **12**. In the shown embodiment the faceplate **10** has five radially extending tongues 15 with concave portions **16** between the tongues **15**. The number of tongues **15**, their length and width can be chosen according to the needs of the particular application, and they need not be identical. The tongues provide good shapeability of the faceplate when attaching it to irregular parts on the skin of a user.

The flange **13** of the faceplate **10** has a generally circular contour except for a radially extending, relatively wide tongue **17**. The carrying sheet **24** of the intermediate sealing member **20** also has a radially extending tongue **25**, which is narrower than the tongue **17** of the flange **13** of the faceplate **10**. When the ostomy appliance is assembled, then the tongues **17** and **25** are free, i.e. they are not adhered to any other of the respective component of the appliance. The tongues 17 and **25** are of different width, which makes it easy for the user to distinguish between them and select the desired one of them for the purpose stated below.

When disassembling the appliance the user can choose whether to change the collection bag **1** only, the bag **1** and the intermediate sealing member **20**, or the whole appliance.

When the user wishes to change the collection bag **1** only, then he or she grips the tongue **25** of the carrier sheet **24** of the intermediate sealing member **20** to retain the intermediate sealing member **20** adhered to the flange **13** of the faceplate **10,** and he then grips and pulls the collection bag **1** and separates it from the carrier sheet **24** of the intermediate sealing member **20**. The faceplate **10** and the intermediate sealing member **20** will then be retained on the peristomal skin of the user.

When the user wishes to change the collection bag **1** and the intermediate sealing member 20, then he or she grips the tongue **17** of the flange **13** of the faceplate **10** to retain only the faceplate **10** adhered to the peristomal skin, and he then grips and pulls the tongue **25** of the carrier sheet 24 of the intermediate sealing member **20** to separate the intermediate sealing member 20 with the collection bag **1** adhered thereto from the flange **13** of the faceplate **10**, which is thus retained on the peristomal skin of the user.

When the user wishes to change the whole appliance, then he or she grips the tongue **17** of the flange **13** of the faceplate **10** and pulls the whole appliance off the skin. Alternatively the user can peel one of the tongues **15** of the faceplate **10** and use it to pull the whole appliance off the skin.

## Claims

1. An ostomy appliance comprising
- a body side member having an adhesive wafer for securing said body side member to a user's skin, said body side member having a body side member opening therein for receiving a stoma of said user when said body side member is secured to said user's skin,
- a collection bag having a bag opening therein and means for securing said collection bag to said body side member with said bag opening in alignment with said body side member opening so as to receive bodily excretions from said stoma,
wherein said adhesive wafer has radially extending tongues with concave contour portions between said tongues.

2. An ostomy appliance according to claim 1 wherein said concave contour portions have an angular extent of substantially the same size as the angular extent of said tongues.

3. An ostomy appliance according to claim 1 wherein said adhesive wafer includes a backing layer and further includes a flange secured to said backing layer at an edge of said body side member opening.

4. An ostomy appliance according to claim 3 wherein said flange includes a free, radially extending tongue not secured to said backing layer.

5. An ostomy appliance according to claim 4 further including a sealing member between said body side member and said collection bag, said sealing member having a carrier sheet with a free, radially extending tongue.

6. An ostomy appliance according to any of the preceding claims further including a collection bag having means for securing said collection bag to said flange.

7. An ostomy appliance according to claim 6 wherein said means for securing said collection bag to said flange includes a releasable adhesive.

8. An ostomy appliance according to claim 6 wherein said means for securing said collection bag to said flange includes a releasable mechanical coupling mechanism.
